Europäisches Patentamt

European Patent Office

Office européen des brevets

(11) Veröffentlichungsnummer: **0 126 792**
**B1**

(12) **EUROPÄISCHE PATENTSCHRIFT**

(45) Veröffentlichungstag der Patentschrift:
15.10.86

(51) Int. Cl.⁴: **C 07 F 7/08,** A 61 L 9/01,
A 61 L 9/04

(21) Anmeldenummer: 83105271.7

(22) Anmeldetag: 27.05.83

(54) Verfahren zur Herstellung von Hexamethylcyclotrisiloxan und eine Verwendung des so hergestellten Cyclotrisiloxans.

(43) Veröffentlichungstag der Anmeldung:
05.12.84 Patentblatt 84/49

(45) Bekanntmachung des Hinweises auf die Patenterteilung:
15.10.86 Patentblatt 86/42

(84) Benannte Vertragsstaaten:
AT BE CH DE FR GB IT LI NL SE

(56) Entgegenhaltungen:
DE-C-875 046
GB-A-774 040
US-A-2 618 648

CHEMICAL ABSTRACTS, Band 91, Nr. 24, 10
December 1979, Seite 363, Nr. 196797k, Columbus,
Ohio, US
Noll, Chemie u. Technologie der Silikone,
Weinheim 1968, S. 201, 195

(73) Patentinhaber: Consortium für elektrochemische
Industrie GmbH, Zielstattstrasse 20, D-8000
München 70 (DE)

(72) Erfinder: Kreuzer, Franz- Heinrich, Dr. Dipl.-
Chem., Josef- Gerstner- Strasse 14d, D-8033
Martinsried (DE)
Erfinder: Gebauer, Helmut, Dr. Dipl.- Chem.,
Schaffhauser Strasse 18/7, D-8000 München 71
(DE)

**Beschreibung**

W. Patnode und D.F. Wilcock beschreibem im Journal of the American Chemical Society, Vol. 68 (1946) Seite 360/361 die Herstellung von Hexamethylcyclotrisiloxan durch Erwärmen von höhermolekularem Dimethylpolysiloxan auf 350°C. Es bestand nun die Aufgabe, Hexamethylcyclotrisiloxan im besserer Ausbeute, als sie bei dem vorstehend erwähnten Verfahren erzielt wird, herzustellen. Diese Aufgabe wird durch die Erfindung gelöst.

Gegenstand der Erfindung ist ein Verfahren zur Herstellung von Hexamethylcyclotrisiloxan durch Depolymerisieren von linearem, verzweigtem oder vernetztem Organopolysiloxan, das mindestens zu 50 Molprozent aus Dimethylsiloxaneinheiten besteht, unter Erwärmen auf mindestens 350°C und gleichzeitigem Abdestillieren der dabei gebildeten cyclischen Dimethylpolysiloxane, dadurch gekennzeichnet, daß dieses Depolymerisieren in Abwesenheit von eine Siloxangruppierung angreifenden Stoffen erfolgt.

Als lineare, verzweigte oder vernetzte Organopolysiloxane, die mindestens zu 50 Molprozent aus Dimethylsiloxaneinheiten bestehen, können auch im Rahmen der Erfindung alle linearen, verzweigten oder vernetzten Organopolysiloxane eingesetzt werden, die mindestens zu 50 Molprozent aus Dimethylsiloxaneinheiten bestehen und aus denen somit auch bisher durch Umlagerung von Siloxangruppierungen cyclische Dimethylpolysiloxane hergestellt werdem konnten. Bevorzugt sind lineare oder praktisch lineare oder vermetzte, mindestens zu 95 Molprozent aus Diorganosiloxaneinheiten bestehende Organopolysiloxane, deren organische Gruppen mindestens zu 95 Molprozent Methylgruppen sind. Die gegebenenfalls zusätzlich zu Methylgruppen in den im Rahmen der Erfindung eingesetzten Organopolysiloxanen vorliegenden anderen organischen Gruppen sind vorzugsweise Ethyl-, Vinyloder Phenylgruppen oder Gemische aus mindestens zwei solcher anderer Gruppen. Die gegebenenfalls zusätzlich zu Diorganosiloxaneinheiten vorliegenden anderen Siloxaneinheiten sind insbesondere Triorganosiloxan-) Monoorganosiloxan oder SiO$_{4/2}$-Einheiten oder Gemische aus mindestens zwei solcher anderer Einheiten.

Bei dem erfindungsgsmäßen Verfahren eingesetztes Organopolysiloxan kann in mehr oder weniger reiner Form, zumindest aber im wesentlicheh frei von bei 350°C eine Siloxangruppierung angreifenden Stoffen, vorliegen. Bei dem erfindungsgemäßen Verfahren eingesetztes Organopolysiloxan kann aber auch z.B. in Form von zu Elastomeren vernetzbaren, teilweise oder vollständig vernetzten Massen, die meist zusätzlich zu vernetzbarem bzw. vernetztem Organopolysiloxan übliche Hilfsstoffe für die Elastomerherstellung, wie Füllstoffs, Pigmente, Vernetzungskatalysatoren, wie organische Zinnverbindungen, bzw. Vernetzungskatalysator-Spaltprodukte, enthalten, vorliegen, solange sie zumindest im wesentlichen frei von bei 350°C eine Siloxangruppierung angreifenden Stoffen sind.

Bei dem erfindungsgemäßen Verfahren kann selbstverständlich nicht nur eine Art von Organopolysiloxan, sondern es können auch Gemische aus verschiedenen Arten von Organopolysiloxanen eingesetzt werden.

Das Erwärmen bei dem erfindungsgemäßen Verfahren wird vorzugsweise bei höchstens 800°C durchgeführt.

Eine Siloxangruppierung, also eime Gruppierung der Formel

■ SiOSi ■

angreifende Stoffe und damit zur Umlagerung, Polymerisation oder Depolymerisation von Organopolysiloxan führend, sind nicht z.B. bei Temperaturen von mindestens 350°C aus z.B. orgamischen Zinnverbindungen gebildete Zinnoxide, wohl aber z.B. bei 500°C beispielsweise die Alkaligehalte von üblichem Geräteglas. Die Anwesenheit von eine Siloxangruppierung angreifenden Stoffen ist nun erfindungsgemäß zu vermeiden. Somit müssen nicht nur die bei dem erfindungsgemäßen Verfahren eingesetzten Orgamopolysiloxane, sondern auch die im Rahmen des erfindungsgemäßen Verfahrens verwendeten Vorrichtungen, soweit ihre Oberfläche eine Temperatur von mindestens 350°C aufweist, im techmisch durchführbaren Ausmaß von Stoffen, welche eine Siloxangruppierung bei 350°C angreifen, frei sein.

Vorzugsweise erfolgt bei dem erfindungsgemäßen Verfahren das Erwärmen von linearem, verzweigtem oder vernetztem Organopolysiloxan, das mindestens zu 50 Molprozent aus Dimethylsiloxaneinheiten besteht, auf mindestens 350°C in Behältern aus Eisen bzw. Stahl, Kupfer, Nickel oder Chrom oder von alkalifreien Legierungen dieser Metalle.

Während des Erwärmens des Organopolgsiloxans auf mindestens 350°C, kann durch dieses Organopolysiloxan ein gegenüber diesem Orgamopolysiloxan inertes Gas, wie Stickstoff, geleitet werden.

Weil dies den geringsten Aufwand erfordert, wird das erfindungsgemäße Verfahren vorzugsweise beim Druck der umgebenden Atmosphäre, also bei 1 bar odsr etwa 1 bar, durchgeführt. Falls erwünscht, können jedoch auch höhers oder niedrigere Drücke angewendet werden.

Falls erwünscht, kann das Erwärmen von linearem, verzweigtem oder vernetztem Organopolysiloxan, das mindestens zu 50 Molprozent aus Dimethylsiloxameinheiten besteht, auf mindestens 350°C unter Rühren oder Umwälzen erfolgen. Das erfindungsgemäßs Verfahren kann absatzweise, halb- oder vollkontinuierlich durchgeführt werden.

Das erfindungsgemäß hergestellts Hexamethylcyclotrisiloxan eignet sich ausgezeichnet als Trägerstoff für Duftstoffe, wobei sich dieser Trägerstoff im Gemisch mit anderen Trägerstoffen wie Tetramethylcyclobutandion, befinden kann.

Die in den folgenden Beispielen und Vergleichsversuchen befindlichen Angaben von Prozentsätzen und Teilen beziehen sich jeweils auf das Gewicht.

Der in den Beispielen erwähnte V4A-Stahl besteht aus 18 Gewichtsprozent Chrom, 11 Gewichtsprozent Nickel, 2 Gewichtsprozent Molybdän, 0,07 Gewichtsprozent Kohlenstoff und Eisen als Rest.

**Beispiel 1 und Vergleichsversuch a) bis d)**

In ein Gefäß aus V4A-Stahl mit einem Innendurchmesser von 90 mm und einer Röhe von 180 mm, das elektrisch auf 600°C beheizt ist, werden von oben gleichzeitig, jedoch durch zwei verschiedene Leitungen, kontinuierlich 160 ml (gemessen bsi Normalbedingungsn) Stickstoff je Minute und 510 ml je Stunde im den endständigen Einheiten je eine Si-gebundene Hydroxylgruppe aufweisendes Dimethylpolysiloxan mit einer Viskosität von 100 mPa.s bei 25°C eingeleitet. In einem auf dem Gefäß angebrachten absteigenden Kühler werden die bei dem Erwärmen gebildeten und abdestillierten cyclischen Diorganopolysiloxane kondensiert.

Bei den Vergleichsversuchen enthält das Gefäß in dem Organopolysiloxan erwärmt wird, zusätzlich zu diesen Organopolysiloxan und Stickstoff, jeweils 0,5 g einer eine Siloxanyruppierung angreifenden Alkaliverbindung, wie in Tabelle 1 angegeben.

Es werden folgende Ergebnisse erhalten:

**Tabelle 1**

| Beispiel bzw. Vergleichs- versuch | Alkaliver- bindung | Menge in % von cyclischem Dimethylpolysiloxan mit fol- gender Anzahl der Siloxaneinheiten | | | | | | Trisiloxan/Tetrasiloxan Gewichtsverhältnis |
|---|---|---|---|---|---|---|---|---|
| | | 3 | 4 | 5 | 6 | 7 | 8 | |
| 1 | — | 65,8 | 26,2 | 5,0 | 1,4 | 1,1 | 0,5 | 2,51 |
| a) | Li$_2$CO$_3$ | 49,6 | 22,4 | 11,2 | 12,9 | 2,8 | 0,8 | 2,21 |
| b) | Na$_2$CO$_3$ | 33,0 | 22,5 | 11,4 | 23,0 | 7,8 | 2,0 | 1,47 |
| c) | K$_2$CO$_3$ | 30,0 | 32,1 | 14,0 | 13,4 | 8,1 | 2,4 | 0,93 |
| d) | CsCO$_3$ | 20,9 | 51,2 | 20,3 | 6,3 | 1,3 | | 0,40 |

**Beispiel 2**

Zur Herstellung eines vernetzten Organopolysiloxans werden 100 Teile eines in den endständigen Einheiten je eine Sigebundene Hydroxylgruppe aufweisenden Dimethylpolysiloxans mit einer Viskosität von 500 mPa-s mit 3 Teilen Ethylpolysilikat mit einem $SiO_2$-Gehalt von 40 %, und 1 Teil Dibutylzinndiacylat, wobei sich die Acyatgruppen jeweils von einem Gemisch aus 9 bis 11 Kohlenstoffatome je Molekül aufweisenden Carbonsäuren, worin die Carboxylgruppe bei mindestens 90 % der Säuren an ein tertiäres Kohlenstoffatom gebunden ist, ableiten (sogenanntem "Dibutylzinndiversatat"), vermacht. Die Masse wird in eine Form gegossen und dort bei Raumtemperatur vernetzen gelassen.

Nach 8 Tagen wird derartig hergestelltes Organopolysiloxanelastomer in der in Beispiel 1 beschriebenen Vorrichtung ohne Zugabe eines weiteren Stoffes auf 500°C erwärmt. Es wird folgendes Ergebnis erhalten:

Menge in % von cyclischen Dimethylpolysiloxanmit folgender Anzahl von Siloxaneinheiten

3 4 5 6 7

73,6 16,2 2,2 1 1

Das Gewichtsverhältnis von Trisiloxan zu Tetrasiloxan beträgt 4,54.

**Beispiele 3 bis 7**

Die für Beispiel 1 beschriebene Arbeitsweise wird wiederholt mit folgenden Abänderungen:

I. Zwischen dem Gefäß, in das das Organopolysiloxan eingebracht und erwärmt wird, und den absteigenden Kühler befindet sich ein Rohr aus Metall, das durch einen Mantel auf 1 35 °C erwärmt wird. Der absteigende Kühler wird bei 80°C gehalten.

II. Die Zugabegeschwindigkeit des Stickstoffs beträgt statt 160 ml je Minute 250 ml je Minute (gemessen bei Normalbedingungen).

III. Bei Beispiel 3 beträgt zwar die Viskosität des Dimethylpolysiloxans ebenfalls 100 mPa-s bei 25°C bei Beispiel 4 bis 7 beträgt sie jedoch jeweils 600 mPa.s bei 25°C.

IV. Die Zugabegeschwindigkeit des eingesetzten Dimethylpolysiloxans hat die in Tabelle 2 angegebenen Werte.

V. Die Temperatur, auf die das eingesetzte Dimethylpolysiloxan erwärmt wird, hat die in Tabelle 2 angegebenen Werte.

**Tabelle 2**

| Beispiel | Tempera-tur* | Dimethyl-polysilox-an | Menge in % von cyclischem Di-methylpolysiloxan mit folgender Anzahl der Siloxaneinheiten | | | Trisiloxan/Tetra-siloxan Gewichts-verhältnis |
|---|---|---|---|---|---|---|
| | °C | ml/h | | | | |
| | | | 3 | 4 | 5 bis 8 | |
| 3 | 600 | 960 | 72,3 | 23,4 | 4,3 | 3,09 |
| 4 | 600 | 360 | 77,8 | 22,2 | 0,1 | 3,52 |
| 5 | 600 | 720 | 78,1 | 21,7 | 0,6 | 3,60 |
| 6 | 515 | 1080 | 80,5 | 18,0 | 1,5 | 4,47 |
| 7 | 505 | 1800 | 78,7 | 18,0 | 3,3 | 4,37 |

* auf die eingesetztes Organopolysiloxan erwärmt wird

### Vergleichsversuche e) bis g)

In einem 1-1-Glaskolben werden 500 g in den endständigen Einheiten je eine Si-gebundene Hydroxylgruppe aufweisendes Dimethylpolysiloxan mit einer Viskosität von 500 mPa.s bei 25°C im Gemisch mit den in der folgenden Tabelle 3 angegebenen Zusätzen auf die in Tabelle 3 angegebenen Temperaturen erwärmt. Über eine etwa 200 mm lange Füllkörperkolonne werden die cyclischen Dimethylpolysiloxane abdestilliert.

**Tabelle 3**

| Vergleichs-versuch | Temperatur °C | Zusatz | Menge in % von cyclischem Dimethylpolysiloxan mit folgender Anzahl der Siloxaneinheiten | | | | | | Trisiloxan/Tetrasiloxan Gewichtsverhältnis |
|---|---|---|---|---|---|---|---|---|---|
| | | | 3 | 4 | 5 | 6 | 7 | 8 | |
| e) | 150 | 15 g KOH | 3,6 | 92,3 | 4,1 | | | | 0,04 |
| f) | 150 | 5 g p-Toluolsulfonsäure | 0,5 | 65,9 | 20,0 | 11,0 | 1,9 | 0,7 | 0,01 |
| g) | 500 | — | 38,7 | 17,8 | 9,1 | 21,5 | 10,5 | 2,27 | 2,17 |

**Patentansprüche**

1. Verfahren zur Herstellung von Hexanethylcyclotrisiloxan durch Depolymerisieren von linearem, verzweigten oder vernetztem Organopolysiloxan, das mindestens zu 50 Molprozent aus Dimethylpolysiloxaneinheiten besteht, unter Erwärmen auf mindestens 350°C und gleichzeitigebdestillieren der da bei gebildeten cyclischen Dimethylpolysiloxane,
dadurch gekennzeichnet,
daß dieses Depolymerisieren in Abwesenheit von eine Siloxangruppierung angreifenden Stoffen erfolgt.

2. Verfahren nach Anspruch 1,
dadurch gekennzeichnet,
daß das Depolymerisieren des Organopolysiloxans in einem Behälter aus Eisen bzw. Stahl, Kupfer, Nickel oder Chrom oder von alkalifreien Legierungen dieser Metalle erfolgt.

**Claims**

1. Process for the manufacture of hexamethylcyclotrisiloxane by depolymerising a linear, branched or crosslinked organopolysiloxane, at least 50 mole per cent of which comprises dimethylpolysiloxane units, while heating to at least 350°C, and simultaneously distilling off the cyclic dimethylpolysiloxanes thereby formed, characterised in that this depolymerisation is carried out in the absence of substances that attack a siloxane grouping.

2. Process according to claim 1, characterised in that the depolymerisation of the organopolysiloxane is carried out in a container made of iron or steel, copper, nickel or chromium, or made of alkali-free alloys of those metals.

**Revendications**

1. Procédé de préparation de l'hexaméthylcyclotrisiloxane par dépolymérisation en chauffant à des température d'au moins 350°C d'un poly-organosiloxane linéaire ramifie ou réticulé, constilué, pour au moins 50 % en moles, de motifs diméthylsiloxanes, et séparation simultanée par distillation, des poly-diméthylsiloxanes cycliques qui se sont alors formés, procédé caractérisé en ce qu'on effectue cette dépolymérisation par voie thermique en l'absence de corps attaquant un groupement siloxanique.

2. Procédé selon la revendication 1, caractérisé en ce que la dépolymérisation du poly-organosiloxane est effectuée dans un récipient en fer ou en acier, en cuivre, en nickel ou en chrome, ou en alliages de ces métaux dépourvus de composés alcalins.